Europäisches Patentamt

**European Patent Office**

Office européen des brevets

(19)

(11) Publication number: **0 215 504**
**B1**

(12)

# EUROPEAN PATENT SPECIFICATION

(45) Date of publication of the patent specification:
05.12.90

(51) Int. Cl.⁵: **C11D 1/37,** C11D 1/06,
C11D 1/29, A61K 7/08

(21) Application number: **86201421.4**

(22) Date of filing: **15.08.86**

(54) Washing and cleansing compositions containing alkyl ether sulphates.

(30) Priority: **16.08.85 NL 8502267**

(43) Date of publication of application:
**25.03.87 Bulletin 87/13**

(45) Publication of the grant of the patent:
**05.12.90 Bulletin 90/49**

(84) Designated Contracting States:
**AT BE CH DE FR GB IT LI NL SE**

(56) References cited:
**EP-A- 0 102 118**

(73) Proprietor: **STAMICARBON B.V., Mijnweg 1, NL-6167 AC
Geleen(NL)**

(72) Inventor: **Smid, Jacob Kornelis, J.G. Heuthorststraat 61,
NL-7009 CL Doetinchem(NL)**
Inventor: **Meijer, Hamke, Catharinadaal 31, NL-6715 KA
Ede(NL)**

ACTORUM AG

## Description

The invention concerns detergent mixtures, containing alkylethersulphates and one or more alkylamido polyglycolether carboxylic acids and/or salts thereof, washing and cleansing agents containing such mixtures and the application of the mixtures and/or washing and cleansing agents.

Alkylethersulphates have been known for a long time as anionic tensides applied in washing and cleansing agents.

They are prepared by first ethoxylating fatty alcohols and then sulphating the obtained ether adducts. In the ethersulphates an $-SO_3H$-group is linked to the O-atoms of the terminal OH-group of the adduct. They are therefore sulphuric acid esters of the adducts. With respect to the corresponding non-sulphated products, they are better tolerated by the skin, but in many cases this tolerance is only limited, which, particularly in the case of washing and/or cleansing preparations coming into close and relatively prolonged contact with the skin, such as shampoos, shower and bath preparations, liquid soap, in particular for washing the hands, and dish-washing preparations for manual dish-washing, is a disadvantage. It is desirable to further improve such preparations in particular, in such a manner that they are better tolerated by the skin. An important aspect with regard to the skin's tolerance is the toxicity of detergent substances or decomposition products thereof with respect to the skin's bacterial flora.

Detergent alkylamido polyglycolether carboxylic acids and salts thereof with favourable toxicity properties are known from the European patent application EP-A 102 118. The so-called secondary toxicity of those compounds is particularly favourable.

An objection to their application, however, is the high cost price.

It is now been discovered that alkylethersulphate-containing detergent mixtures containing 5–20 wt.%, calculated with respect to the total amount of alkylethersulphate and alkylamido polyglycolether carboxylic acid and/or salts thereof, of one or more alkylamidopolyglycolethercarboxylic acids or salts thereof with the formula $R-CO-NH-(C_2H_4O)_n-CH_2COOM$, in which R represents an alkyl group with 5–21 carbon atoms and M a hydrogen atom, an alkali metal, an alkaline earth metal equivalent or an ammonium or amine group and n a number with a value ranging from 2 to 10, have extremely favourable toxicity properties and are well tolerated by the skin at the mentioned low concentrations of the last-mentioned, i.e. most expensive, component. Alkylethersulphates always consist of mixtures of compounds with different degrees of ethoxylation, i.e. they contain varying numbers of glycol groups, often even including a non-ethoxylated fraction.

Within the framework of the description of the invention under consideration, "an alkylethersulphate" is also understood to mean a mixture of compounds with different alkyl groups. In general, the fatty alcohols used to prepare the alkylethersulphates are mixtures. Even if a fatty alcohol with one and the same number of carbon atoms is used, this often consists of a mixture of isomers.

Preferably, alkylamidopolyglycolethercarboxylic acids and/or salts thereof are used in which R in the above formula represents an alkyl group with 11–17 carbon atoms.

The mixtures described in the invention under consideration are therefore quite suitable for washing and cleansing agents coming into close and relatively prolonged contact with the skin, such as shampoos, shower and bath preparations and dishwashing agents.

As alkylethersulphate all compounds usual for this purpose may be applied. A trade product often applied in shampoos and the like is a laurylethersulphate, in which the lauryl radical may, to a maximum of approx. 40%, be replaced by myristyl, the number of glycol units generally being two or three in this product. It will be clear to any specialist that whenever there is mention of a certain number of glycol units in ethoxylated compounds, an average value is implied. It is common knowledge that mixtures of compounds with different numbers of glycol groups are obtained in ethoxylation processes.

In the following examples the invention will be illustrated on the basis of the application of the above-mentioned product, solely because this is the most common trade product.

Obviously the invention is not limited to this. This laurylethersulphate is usually applied as sodium salt, but naturally other salts, such as potassium, magnesium and amine salts, though more expensive, may also be used.

The preparation of the alkylamidopolyglycolethercarboxylic acids is described in the above-mentioned European patent application EP-A-102118.

The following, non-limiting experiments will illustrate the invention.

The experiments were conducted according to the so-called 'patchtest method'. The aim was to determine the degree of primary irritation of the skin caused by the substances concerned in comparison with a control substance. The experiment was conducted with a group of 25 men and women varying in age from 16 to 65.

The samples control substances were applied to the skin of the upper arm by means of a sealing 'finn chamber'. This is a sealing aluminium disc providing a test area of 50 mm² with a diameter of 8 mm and a volume of 25 μl. These are set at standard intervals of 1 cm on a non-sealing support plaster. These plasters were left in contact with the skin for 24 hours and were then removed. The skin reaction was determined one hour after the removal of the plaster. Immediately after this determination a second identical plaster with the same substance was applied to the same part of the skin and then left in contact with the skin for another 24 hours. Once more the reaction was determined one hour after the removal of the plaster. The samples were distributed arbitrarily among the different volunteers. All determinations were performed under standard light conditions by a trained person who did not know which samples had been applied to which persons.

The degree of irritation is expressed in figures from 0 to 4. These figures have the following meaning:

0 = no visible reaction;
1 = reaction only just visible;
2 = slight reaction;
3 = moderate reaction;
4 = serious reaction.

The irritation symptoms were rated as follows:

| Symptom | Abbreviation | Rating |
|---|---|---|
| Vesicles | V | 5 |
| Oedema | Oe | 4 |
| Redness | R | 3 |
| Flaking | F | 2 |
| Dryness | D | 1 |
| Wrinkles | W | 1 |
| Semi-transparency | T | 1 |
| Glasslike | G | 1 |

A rating for each skin area was obtained by multiplying the rating for the degree of reaction by the rating for the observed symptoms. The obtained values were summed to produce a total rating for the degree of irritation in that area.

As an example it can be mentioned that the total score for an area where R3, D2 and G1 are observed, would be:

$(3 \times 3) + (2 \times 1) + (1 \times 1) = 12.$

The substances examined were laurylpolyethersulphate as sodium salt with 2 ethoxy groups, indicated as 'A' below, and lauryl-NH-$(OC_2H_4)_xOCH_2COONa$, in which x equals 4, indicated as 'B' below.

Experiments were conducted with respectively

1. 100% A
2. 95% A + 5% B
3. 88% A + 12% B
4. 100% B.

All samples were examined in concentrations of 15% in water. The following table shows the average results of the ratings of the skin irritation after 24 and 48 hours for each of these experiments.

| Sample | Average rating for the skin irritation after | |
|---|---|---|
| | 24 hours | 48 hours |
| 1. | 1.79 | 4.58 |
| 2. | 1.75 | 4.04 |
| 3. | 1.58 | 3.88 |
| 4. | 1.52 | 1.95 |

It is apparent from the table that by using relatively large amounts of relatively cheap alkylethersulphates for detergent mixtures as described in the invention, a product that is tolerated very well (better than expected) by the skin is nevertheless obtained.

3

The invention also concerns the application of the mixtures under consideration and the preparations containing the mixtures under consideration in commerce and industry, e.g. in hairdressing salons, the catering industry and the like.

**Claims for the Contracting States: BE, CH, DE, FR, GB, IT, LI, NL, SE**

1. Detergent mixture containing alkylethersulphate and one or more alkylamido polyglycolether carboxylic acids and/or salts thereof, characterized in that the mixture contains one or more alkylamidopolyglycolether carboxylic acids and/or salts thereof with the formula $R-CO-NH-(C_2H_4O)_n-CH_2-COOM$, in which R represents an alkyl group with 5–21 carbon atoms and M a hydrogen atom, an alkali metal atom, an alkaline earth metal equivalent, an ammonium or amine group and n a number with a value ranging from 2 to 10, in an amount of 5–20% (wt) calculated with respect to the total amount of alkylether sulphate and alkylamidopolyglycolether carboxylic acid and/or salts thereof.

2. Compound as described in claim 1, characterized in that it contains an alkylamidopolyglycolether carboxylic acid and/or salt thereof with the above formula, in which R represents an alkyl group with 11–17 carbon atoms.

3. Compound as described in claim 1 or 2, characterized in that it contains sodiumlaurylethersulphate with 2 glycol groups as alkylethersulphate.

4. Washing and/or cleansing agent containing a compound as described in one or more of claims 1–3.

5. Application of a compound as described in one or more of claims 1–3 and/or of a washing and/or cleansing agent as described in claim 4 in commerce and industry.

**Claims for the Contracting State: AT**

1. Method for the preparation of a detergent mixture, by mixing alkylethersulphate and one or more alkylamido polyglycolether carboxylic acids and/or salts thereof, characterized in that one or more alkylamidopolyglycolether carboxylic acids and/or salts thereof with the formula $R-CO-NH-(C_2H_4O)_n-CH_2COOM$, in which R is an alkyl group with 5–21 carbon atoms and M a hydrogen atom, an alkali metal atom, an alkaline earth metal equivalent or an ammonium or amine group and n a number with a value ranging from 2 to 10, are added in an amount of 5–20% (wt) calculated with respect to the total amount of alkylethersulphate and alkylamidopolyglycolether carboxylic acid and/or salts thereof.

2. Method according to claim 1, characterized in that an alkylamidopolyglycolether carboxylic acid and/or salt thereof with the above formula, in which R is an alkyl group with 11–17 carbon atoms, is used.

3. Method according to claim 1 or 2, characterized in that the alkylethersulphate used is sodiumlaurylethersulphate with 2 glycol groups.

**Patentansprüche für die Vertragsstaaten: BE, CH, DE, FR, GB, IT, LI, NL, SE**

1. Detergensmischung enthaltend Alkyläthersulfat und eine oder mehrere Alkylamidopolyglykoläthercarbonsäuren und/oder Salze hievon, dadurch gekennzeichnet, daß die Mischung eine oder mehrere Alkylamidopolyglykoläthercarbonsäuren und/oder Salze hievon der Formel $R-CO-NH-(C_2H_4O)_n-CH_2COOM$, worin R eine Alkylgruppe mit 5 bis 21 Kohlenstoffatomen bedeutet, M ein Wasserstoffatom, ein Alkalimetallatom, ein Erdalkalimetalläquivalent oder eine Ammonium- oder Amingruppe darstellt und n eine Zahl mit einem Wert von 2 bis 10 ist, in einer Menge von 5 bis 20 % (Gew.), bezogen auf die Gesamtmenge an Alkyläthersulfat und Alkylamidopolyglykoläthercarbonsäure und/oder Salzen hievon, enthält.

2. Mischung, wie in Anspruch 1 beschrieben, dadurch gekennzeichnet, daß sie eine Alkylamidopolyglykoläthercarbonsäure und/oder ein Salz hievon der obigen Formel, worin R eine Alkylgruppe mit 11 bis 17 Kohlenstoffatomen darstellt, enthält.

3. Mischung, wie in Anspruch 1 oder 2 beschrieben, dadurch gekennzeichnet, daß sie Natriumlauryläthersulfat mit 2 Glykolgruppen als Alkyläthersulfat enthält.

4. Wasch- und/oder Reinigungsmittel enthaltend eine Mischung, wie in einem oder mehreren der Ansprüche 1 bis 3 beschrieben.

5. Verwendung einer Mischung, wie in einem oder mehreren der Ansprüche 1 bis 3 beschrieben, oder eines Wasch- und/oder Reinigungsmittels, wie in Anspruch 4 beschrieben, im Gewerbe und in der Industrie.

**Patentansprüche für den Vertragsstaat: AT**

1. Verfahren zur Herstellung einer Detergensmischung durch Mischen von Alkyläthersulfat und einer oder mehrerer Alkylamidopolyglykoläthercarbonsäuren und/oder Salze hievon, dadurch gekennzeichnet, daß eine oder mehrere Alkylamidopolyglykoläthercarbonsäuren und/oder Salze hievon der Formel $R-CO-NH-(C_2H_4O)_n-CH_2COOM$, worin R eine Alkylgruppe mit 5 bis 21 Kohlenstoffatomen bedeutet, M ein Wasserstoffatom, ein Alkalimetallatom, ein Erdalkalimetalläquivalent oder eine Ammonium- oder Amingruppe darstellt und n eine Zahl mit einem Wert von 2 bis 10 ist, in einer Menge von 5 bis 20%

(Gew.), bezogen auf die Gesamtmenge an Alkyläthersulfat und Alkylamidopolyglykoläthercarbonsäure und/oder Salzen hievon, zugesetzt werden.

2. Verfahren nach Anspruch 1, dadurch gekennzeichnet, daß eine Alkylamidopolyglykoläthercarbonsäure und/oder ein Salz hievon der obigen Formel, worin R eine Alkylgruppe mit 11 bis 17 Kohlenstoffatomen darstellt, verwendet wird.

3. Verfahren nach Anspruch 1 oder 2, dadurch gekennzeichnet, daß das verwendete Alkyläthersulfat Natriumlauryläthersulfat mit 2 Glykolgruppen ist.

**Revendications pour les Etats contractants: BE, CH, DE, FR, GB, IT, LI, NL, SE**

1. Mélange détergent contenant un alkyléthersulfate et un ou plusieurs acide(s) alkylamidopolyglycoléthercarboxylique(s) et/ou leurs sels, caractérisé en ce que le mélange contient un ou plusieurs acide(s) alkylamidopolyglycoléthercarboxylique(s) et/ou leurs sels répondant à la formule:
$$R-CO-NH-(C_2H_4O)_n-CH_2COOM$$
dans laquelle R représente un radical alkyle de 5 à 21 latomes de carbone et M représente un atome d'hydrogène, un atome de métal alcalin, un équivalent de métal alcalino-terreux, un radical ammonium ou amine et n est un nombre compris entre 2 et 10, en une proportion de 5 à 20% en poids par rapport à la quantité totale de l'alkyléthersulfate et de l'acide alkylamidopolyglycoléthercarboxylique et/ou des sels de celui-ci.

2. Composé selon la revendication 1, caractérisé en ce qu'il contient un acide alkylamidopolyglycoléthercarboxylique et/ou un sel de celui-ci répondant à la formule ci-dessus dans laquelle R représente un radical alkyle de 11 à 17 atomes de carbone.

3. Composé selon la revendication 1 ou 2, caractérisé en ce qu'il contient un lauryléthersulfate de sodium avec deux groupes glycol à titre d'alkyléther sulfate.

4. Agent de lavage et/ou de nettoyage contenant un composé tel que défini dans une ou plusieurs des revendications 1 à 5.

5. Application d'un composé décrit dans une ou plusieurs des revendications 1 à 3 et/ou d'un agent de lavage et/ou de nettoyage comme décrit dans la revendication 4, au commerce et à l'industrie.

**Revendications pour l'Etat contractant: AT**

1. Procédé de préparation d'un mélange détergent consistant à mélanger un alkyléthersulfate et un ou plusieurs acide(s) alkylamidopolyglycoléthercarboxylique(s) et/ou leurs sels, caractérisé en ce qu'on introduit un ou plusieurs acide(s) alkylamidopolyglycoléthercarboxylique(s) et/ou leurs sels répondant à la formule:
$$R-CO-NH-(C_2H_4O)_n-CH_2COOM$$
dans laquelle R est un radical alkyle de 5 à 21 atomes de carbone et M est un atome d'hydrogène, un atome de métal alcalin, un équivalent de métal alcalino-terreux ou un radical ammonium ou amine et n est un nombre de 2 à 10, en une quantité de 5 à 20% en poids calculée par rapport au total de l'alkyléthersulfate et de l'acide alkylamidopolyglycoléthercarboxylique et/ou des sels de celui-ci.

2. Procédé selon la revendication 1, caractérisé en ce qu'on utilise un acide alkylamidopolyglycoléthercarboxylique et/ou un sel de celui-ci répondant à la formule ci-dessus dans laquelle R est un radical alkyle de 11 à 17 atomes de carbone.

3. Procédé selon la revendication 1 ou 2, caractérisé en ce que l'alkyléthersulfate utilisé est le lauryléthersulfate de sodium avec deux groupes glycol.